# EUROPEAN PATENT SPECIFICATION

(11) **EP 2 353 633 B1**
(45) Date of publication and mention of the grant of the patent: **06.04.2016**
(21) Application number: 10153051.7
(22) Date of filing: 09.02.2010
(51) Int. Cl.: A61M 25/10

(54) **Balloon catheter for post conditioning**
Ballonkatheter zur Postkonditionierung
Cathéter à ballonnet pour post-conditionnement

(43) Date of publication of application: 10.08.2011
(73) Proprietor: Sanofi-Aventis Deutschland GmbH, 65929 Frankfurt am Main (DE)
(72) Inventor: Gille, Andreas, 3032 Ascot Vale, Victoria (AU); Schäfer, Matthias, 65926 Frankfurt am Main (DE)

(56) References cited:
- WO-A1-94/02193
- WO-A1-2007/067226
- US-A- 5 549 551
- US-A1- 2006 100 639
- US-A1- 2006 200 191

## Description

The present invention relates to a medical device for use in post conditioning treatment.

In particular, the present invention relates to a catheter with expandable balloons for use in the early post conditioning in angioplasty like treatment, especially for reopening a constricted portion generated in a body tissue or body lumen, such as a blood vessel.

Myocardial infarction (MI) or acute,myocardial infarction (AMI), commonly known as a heart attack is the interruption of blood supply to part of the heart, commonly due to occlusion of a coronary artery. Percutaneous transluminal angioplasty, particularly coronary angioplasty using balloon catheters, has been developed as an alternative to bypass surgery for revascularization of stenotic and occluded body vessels, such as coronary arteries. In the standard angioplasty procedure the balloon catheter is positioned in the affected vessel so that by inflating the balloon multiple times the desired dilation of the occluded segment can be achieved. After the desired result of reopening the vessel has been obtained by the balloon inflations the balloon is completely deflated and the balloon catheter is withdrawn. Prompt restoration of blood flow to ischemic tissue limits infarct size. But the process of restoring the blood flow to the ischemic tissue, especially the myocardium, however, can also induce injury. This phenomenon, termed reperfusion injury, can paradoxically reduce the beneficial effects of the reperfusion. Reperfusion can also result in additional damage and complications. Post conditioning treatment in the early stage of reperfusion (<15 min) is known to reduce reperfusion injury. The known post conditioning treatment includes sequential/alternately manually controlled complete dilation and complete deflation of the balloon in the affected body lumen. Also between the phases of dilatation for reopening the stenotic, occluded segment, already reperfusion occurs. Common dilatation balloons are not suitable for controlling the reperfusion flow and regulating the desirable flow restriction. Additionally with the manually controlled inflation and deflation of the dilatation balloon it is almost impossible to control the flow rate of the blood and to prevent or reduce reperfusion injury.

WO 2007/067226 discloses a catheter for carotid artery dilatation that includes a multi-chamber balloon assembly mounted at the distal end of the elongated tube that has at least two tandem chambers. Each chamber communicates with a separate lumen. In one embodiment the balloon material may be different from one chamber to the next.

US 2006/0200191 discloses a method and apparatuses for treating an intravascular occlusion comprising two or more separate catheters each equipped with a balloon which can be inserted into each other. A dilatation balloon can be formed of a non-elastic material and the other balloon can be formed of an elastic material.

US 2006/100639 relates to a method for reducing reperfusion injury after therapeutic reperfusion of an infart of a heart or other organ. It also relates to percutaneous transluminal coronary angioplasty PTCA catheters for angioplasty and protection of patients during transcatheter reperfusion therapies.

It is an objective of the present invention to provide an improved catheter adapted for use in post conditioning treatment to at least reduce or prevent reperfusion injury.

In order to achieve this objective, the present invention provides a catheter adapted for use in post conditioning treatment, said catheter comprising:
an elongate tubular body with a proximal and a distal end, at least one dilatation balloon disposed on and securely attached to the exterior of the tubular body, means for inflating and/or deflating said dilatation balloon comprising a first lumen in the tubular body and at least one regulating balloon disposed proximal and/or distal to the dilatation balloon and means for inflating and/or deflating the second balloon comprising a second lumen in the tubular body wherein the catheter comprises at least one flow sensor positioned distal from the balloons and wherein the regulating balloon is made of a different material than the dilatation balloon and the material of the regulating balloon is more elastic than the material of the dilatation balloon.

The elongate tubular catheter body includes a wall which surrounds a plurality of interior lumens. In particular, separate lumens are connected to the interior of the different dilatation and regulating balloons for separate and independent inflating and deflating thereof. Thus, a first lumen is fluidly connected with the dilatation balloon and a fluid or gas is passed through said first lumen to inflate as well as deflate the dilatation balloon. A second lumen terminates in the regulating balloon for separate and independent inflation and deflation thereof.

The tubular body according to the invention is preferably made from polymer materials, such as polyester, polyurethanes, polyamides, polyamide mixtures and copolymers, polyethylene terephthalate and polyethylene mixtures and copolymers.

The term "distal end" according to the present invention shall mean the end of the tubular catheter body, which is closest to the insertion end of the catheter. The term "proximal) end" according to present invention shall mean the end of the tubular catheter body which is furthest away from the insertion end of the device. Accordingly, the term "distal" or "distal direction" is in the direction which-is directed from the proximal end to the distal end of the catheter and the term "proximal" or "proximal direction" is in the direction which is directed from the distal end to the proximal end of the device or a component of the device.

It is in accordance with the invention that the different lumens inside the tubular catheter body can terminate in separate proximal ends of the catheter and may be connected to different devices, such as pumps, pressure measuring and/or other regulation devices.

The dilatation balloon disposed on the tubular catheter body is preferably an angioplasty balloon. The dilatation balloon is preferably made from a non-elastomeric thin walled polymer material such as, polyvinylchloride, polyethylene, various derivatives thereof or other suitable film-forming material, capable of withstanding pressures of ≥ 100 psi, or ≥ 150 psi, without being damaged and without significant or undue stretch or deformation when formed and inflated into the thin-walled balloon. Suitable materials are generally known to those skilled in the art of angioplasty. The low- or even non-compliant material of the dilatation balloon provides for a stable balloon expansion even under high inflation pressure.

The regulating balloon is also disposed on the exterior of the tubular body spaced apart and proximal and/or distal from the dilatation balloon. Thus, it is also within the scope of the present invention that the catheter comprises at least one regulating balloon disposed on the tubular body proximal and at least one regulating balloon distal from the dilatation balloon. According to the invention each balloon is connected to a separate lumen in the interior of the tubular catheter body for separate and independent inflation and deflation.

The regulating balloon according to the invention is made from a more elastic material than the dilatation balloon, such as silicone, polyurethane, a latex material or rubber. The elastic material of the invention gives a good correlation between the inflation pressure and the balloon extension. Therefore inflation and deflation of the regulating balloon according to the invention correlates or can exactly be adapted to a predetermined inflation pressure and vice versa. Thus, advantageously the inflation and deflation of the regulating balloon can be easily and accurately controlled by regulating the inflation pressure of said balloon.

With the regulating balloon series of sequential inflation and deflation of the in a predetermined and/or beneficial extent resulting in full sealing of the vessel (inflation) or reduced low pressure blood flow (partially inflated/deflated) finally resulting in a fully restored blood flow can accurately be performed and chosen by the medical person to fit to the needs of the patient. Like that, the blood flow in the treated vessel behind the regulating balloon can be controlled during the whole treatment. Optimal blood flow and blood pressure in the targeted area in risk of reperfusion injury can be achieved by adjusting the inflation and deflation series of the regulating balloon.

According to an aspect of the invention a catheter is provided wherein by inflation and deflation of the regulating balloon a flow of a fluid is continuously adaptable. This may advantageously be used to continuously regulate the blood flow between a predetermined minimum and a maximum blood flow in the dilatation procedure and post conditioning treatment. The risk of reperfusion injury occurring during both balloon dilatation cycles and post conditioning treatment of the angioplasty procedure by restoration of the blood flow, can be reduced or even prevented.

Ischemia-reperfusion injuries are also a common complication in organ transplantation which occurs when blood supply to the transplanted tissues or organs is temporally interrupted and then reintroduced again (reperfusion). In one example of the invention the flow of a fluid can also be the blood flow in the reperfusion of transplanted organs or tissue and can advantageously be controlled and regulated continuously in a secure and smoothly manner. Also in this case reperfusion injury can be reduced or even avoided. Examples of tissue or organ which might be prevented to suffer an ischemic-reperfusion injury include, but are not limited to heart, kidney, pancreas, liver, brain, and lung.

According to another aspect of the invention, a catheter is provided, comprising at least one pressure sensor for sensing the inflation pressure of the dilatation balloon and/or the regulating balloon and at least one flow sensor.

The pressure sensor can be chosen from sensors well known in the art. As an example, invasive blood pressure sensors and blood pressure transducers can be implemented.

The flow sensor can also be chosen from sensors well known in the art. For example, electromagnetic catheter blood flow meters or invasive catheter flow sensors can be used. In particular, a silicon flow sensor with integrated electronics for invasive blood-flow measurement can be integrally provided on the catheter.

The pressure sensor for sensing the inflation pressure of the balloons may be positioned inside the particular regulating and/or dilatation balloon and/or on the exterior of the regulating and/or dilatation balloon. Controlling the pressure of the balloon at the actual site of the treatment can give vital and more accurate information about the pressure applied to the vessel. Accordingly, with a pressure sensor for measuring the balloon inflation pressure directly placed inside the balloon or on its surface there is given a possibility to control and adjust the inflation and/or deflation of the balloon specifically according to the need of the patient. Furthermore, by positioning the pressure sensor on the surface of the balloon it is advantageously possible to sense the maximum inflation of the balloon in view of the diameter of the targeted vessel without the risk of further injuring the vessel by over-expansion.

The pressure sensors may also be located in the first and/or second lumen of the catheter connected to the respective balloons. The sensing and the possible monitoring of the pressure, particularly the sensing of the actual and accurate inflation pressures of the balloon, and the sensing of a flow of a fluid, allows for an even more exact regulation of the blood flow, dependent on the inflation pressure applied to the regulating balloon. Especially with additional pressure sensors on the exterior of the balloons the actual pressure exerted on the affected vessel wall or tissue may be accurately measured. This provides additional advantages, such as the risk of additional injury of the vessel or tissue by over-expansion of the regulating and/or dilatation balloon can be reduced.

According to the invention at least one flow sensor is positioned distal from the balloons. Like that, the blood flow in the treated vessel behind the balloon can be controlled during the whole treatment. Optimal blood flow and blood pressure in the targeted area in risk of reperfusion injury can be achieved by adjusting pre-programmed inflation and deflation series of the balloon in accordance with the actual measurements of the sensor.

According to an embodiment of the invention the catheter comprises a controlling device for controlling the inflation or deflation of the dilatation balloon and/or the regulating balloon in relation to the sensed flow of a fluid and/or in relation to the sensed pressure. This facilitates achieving the predetermined inflation and deflation pressures of the balloons. Moreover the controlling device allows for an even more exact regulation of a fluid flow dependent on the inflation pressure applied to the regulating balloon. In particular, the fluid flow is the blood flow. Thus, with the use of a catheter according to the invention a perfusion injury in angioplasty treatment, especially between the dilatation phases and in the final restoring of the blood flow by the use of post conditioning treatment, can be reduced or even avoided.

In yet another aspect of the invention the means for inflating and/or deflating the regulating balloon comprise an automatically controllable pump. The automatically controllable pump may regulate the output pressure and may provide a defined fluid flow through the second lumen into the regulating balloon thereby exactly and securely regulating the balloon inflation and deflation. Thereby the blood flow flowing into the ischemic segments can be controlled.,The catheter of the invention allows for an improved perfusion procedure with a reduced risk of perfusion injury.

The controllable pump is preferably controlled in relation to the sensed inflation pressure of the regulating balloon. Signals from sensors can be transmitted to a control or a processor that in turn displays and/or controls the inflation or deflation of the balloon by influencing the inflation/deflation means, such as the controllable pump. The transmittal of sensor signals can be achieved via thin electric or fibre optic wires. The wires can be placed inside the catheter and terminate either in an external control unit or in a control unit integrated in the catheter itself. Alternatively, the sensor signals can be transmitted by wireless communication with a control unit.

Thus it is also in accordance with the invention that the controllable pump is controlled in relation to a sensed fluid flow, particularly the blood flow, alone or in combination with a relation to the sensed inflation pressure of the regulating and/or the dilatation balloon.

According to another aspect of the invention the balloon catheter comprises means for delivery of a drug according to any of the embodiments described above. The means for delivery of a drug can be provided for dispensing or administering a pharmaceutical formulation comprising an active compound, for instance, suitable for treatment of stenotic tissue.

The means for the local delivery of a drug according to one aspect of the invention comprise a separate lumen in the catheter and/or a coating on the balloon and/or a delivery via pores in the balloon. The possible local drug delivery may be used to treat the occluded and reopened segment. The separate lumen may be an infusion lumen, which may terminate in an infusion port or which may be suitably located in the tubular body for the local delivery of a drug. A suitable position can be between a regulating and a dilatation balloon. With both balloons inflated it is possible to achieve an exact volume inside the vessel for a restricted and targeted treatment with special drugs. This is also possible with one regulating balloon positioned proximal and one regulating balloon placed distal from the dilatation balloon. Additionally in this embodiment the dilatation balloon may also be partially or completely deflated and also the area of the occluded, stenotic and reopened segment may be treated. At the same time in the mentioned embodiments it is possible to control the application time of the drug as well as the subsequent transport of the drug downstream when the balloons are deflated. Thus, when a combination of an angioplasty procedure and a local drug therapy is necessary or beneficial the balloon catheter of the present invention can reduce the time of the procedure and is cost saving.

The invention will now be described, by way of example only, with reference to the accompanying drawings, in which:

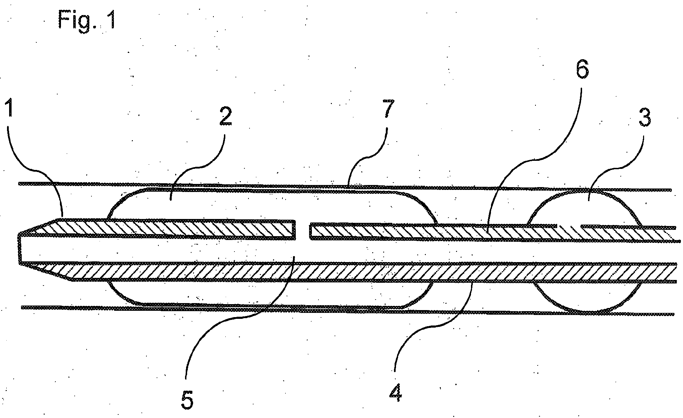

Figure 1 illustrates a schematic sectional view of one embodiment of a catheter according to the present invention,

Figure 1 shows a schematac sectional view of one embodiment of a catheter 1 according to the present invention comprising one dilatation balloon 2 and one regulating balloon 3 on a tubular body 4. Separate lumens 5, 6 interior of said tubular body 4 are connected to the interior of the different dilatation and regulating balloons 2, 3 for separate and independent inflating and deflating thereof. Thus, a first lumen 5 is fluidly connected with the dilatation balloon 2 and a fluid or gas is passed through said first lumen to inflate as well as deflate the dilatation balloon 2. A second lumen 6 terminates in the regulating balloon 3 for separate and independent inflation and deflation thereof. The regulating balloon 3 is made from a different and more elastic material than the dilatation balloon 2. The material of the regulating balloon 3 may be selected from silicone, polyurethane, a latex material or rubber and gives a good correlation between the exerted inflation pressure and the resulting balloon extension. With the regulating balloon 3 series of sequential inflation and deflation of the in a predetermined and/or beneficial extent can accurately be performed resulting in full sealing of the vessel (inflation) or reduced low pressure blood flow (partially inflated /deflated) finally resulting in a fully restored blood flow. In the illustrated embodiment the regulating balloon (3) is positioned in proximal direction of the dilatation balloon (2). Thus, with the use of a catheter (1) according to the invention a reperfusion injury in angioplasty treatment, especially between the dilatation phases and in the final restoring of the blood flow with the use of post conditioning treatment, can be reduced or even avoided.

## Claims

1. A catheter (1) adapted for use in post conditioning treatment, said catheter (1) comprising:
an elongate tubular body (4) with a proximal and a distal end at least one dilatation balloon (2) disposed on the exterior of the tubular body (4)
means for inflating and/or deflating said dilatation balloon (2) comprising a first lumen (5) and
at least one regulating balloon (3) disposed proximal and/or distal to the dilatation balloon (2) and
means for inflating and/or deflating the regulating balloon (3) comprising a second lumen (6) **characterized in that**
the catheter comprises at least one flow sensor positioned distal from the balloons
and the regulating balloon (3) is made of a different material than the dilatation balloon (2) and the material of the regulating balloon (3) is more elastic than the material of the dilatation balloon (2).

2. The catheter (1) according to claim 1 **characterized in that** by inflation of the regulating balloon (3) a flow of a fluid is continuously adaptable.

3. The catheter (1) according to claim 1 or 2 **characterized in that** the catheter (1) comprises at least one pressure sensor for sensing the inflation pressure of the dilatation balloon and/or the regulating balloon.

4. The catheter (1) according to one of the preceding claims **characterized in that** said catheter comprises a controlling device for controlling the inflation or deflation of the dilatation balloon (2) and/or the regulating balloon (3) in relation to the sensed flow of a fluid and/or in relation to the sensed pressure.

5. The catheter (1) according to one of the preceding claims **characterized in that** the means for inflating and/or deflating the regulating balloon (3) comprise a controllable automatically driven pump.

6. The catheter (1) according to claim 5 **characterized in that** the pump is controlled in relation to the sensed inflation pressure of the regulating balloon.

7. The catheter (1) according to one of the preceding claims **characterized in that** the catheter comprises means for a local delivery of a drug.

8. The catheter (1) according to claim 7 **characterized in that** the means for a local delivery of a drug comprise a separate lumen in the tubular body or a coating on the balloon or a delivery via pores in the balloon.

## Patentansprüche

1. Katheter (1), geeignet für die Verwendung zur Postkonditionierungs-Behandlung, wobei der Katheter (1) umfasst:
einen langgestreckten rohrförmigen Körper (4) mit einem proximalen Ende und einem distalen Ende
mindestens einen Dilatationsballon (2), der an der Außenseite des rohrförmigen Körpers (4) angeordnet ist
Mittel zum Aufblasen und/oder Entleeren des Dilatationsballons (2), umfassend ein erstes Lumen (5) und
mindestens einen regulierenden Ballon (3), der proximal und/oder distal zu dem Dilatationsballon (2) angeordnet ist und
Mittel zum Aufblasen und/oder Entleeren des regulierenden Ballons (3), umfassend ein zweites Lumen (6), **dadurch gekennzeichnet, dass**
der Katheter mindestens einen Durchflusssensor aufweist, der distal von den Ballons positioniert ist,
und der regulierende Ballon (3) aus einem anderen Material als der Dilatationsballon (2) hergestellt ist und das Material des regulierenden Ballons (3) elastischer ist als das Material des Dilatationsballons (2).

2. Katheter (1) nach Anspruch 1, **dadurch gekennzeichnet, dass** durch das Aufblasen des regulierenden Ballons (3) ein Durchfluss eines Fluids kontinuierlich anpassbar ist.

3. Katheter (1) nach Anspruch 1 oder 2, **dadurch gekennzeichnet, dass** der Katheter (1) mindestens einen Drucksensor zum Erfassen des Aufblasdrucks des Dilatationsballons und/oder des regulierenden Ballons aufweist.

4. Katheter (1) nach einem der vorhergehenden Ansprüche, **dadurch gekennzeichnet, dass** der Katheter eine Steuervorrichtung zum Steuern des Aufblasens oder Entleerens des Dilatationsballons (2) und/oder des regulierenden Ballons (3) in Bezug auf den erfassten Durchfluss von einem Fluid und/oder in Bezug auf den erfassten Druck aufweist.

5. Katheter (1) nach einem der vorhergehenden Ansprüche, **dadurch gekennzeichnet, dass** die Mittel zum Aufblasen und/oder Entleeren des regulierenden Ballons (3) eine steuerbare, automatisch angetriebene Pumpe aufweisen.

6. Katheter (1) nach Anspruch 5, **dadurch gekennzeichnet, dass** die Pumpe in Bezug auf den erfassten Aufblasdruck des regulierenden Ballons gesteuert ist.

7. Katheter (1) nach einem der vorhergehenden Ansprüche, **dadurch gekennzeichnet, dass** der Katheter Mittel für eine lokale Abgabe eines Arzneimittels aufweist.

8. Katheter (1) nach Anspruch 7, **dadurch gekennzeichnet, dass** die Mittel zur lokalen Abgabe eines Arzneimittels ein separates Lumen in dem rohrförmigen Körper oder eine Beschichtung auf dem Ballon oder eine Abgabe über Poren in dem Ballon umfassen.

## Revendications

1. Cathéter (1) adapté pour être utilisé dans un traitement de post-conditionnement, ledit cathéter (1) comprenant:
un corps tubulaire allongé (4) présentant une extrémité proximale et une extrémité distale,
au moins un ballonet de dilatation (2) qui est disposé sur l'extérieur du corps tubulaire (4),
des moyens pour gonfler et/ou dégonfler ledit ballonet de dilatation (2), comprenant une première lumière (5), et
au moins un ballonet de régulation (3) qui est disposé à proximité et/ou à distance du ballonet de dilatation (2), et
des moyens pour gonfler et/ou dégonfler le ballonet de régulation (3), comprenant une deuxième lumière (6),
**caractérisé en ce que**:
le cathéter comprend au moins un capteur d'écoulement qui est positionné distale des ballonets, et
le ballonet de régulation (3) est constitué d'une matière différente de celle constituant le ballonet de dilatation (2), et la matière constituant le ballonet de régulation (3) est plus élastique que la matière constituant le ballonet de dilatation (2).

2. Cathéter (1) selon la revendication 1, **caractérisé en ce qu'**en gonflant le ballonet de régulation (3) un écoulement de fluide peut être adapté de façon continue.

3. Cathéter (1) selon la revendication 1 ou 2, **caractérisé en ce que** le cathéter (1) comprend au moins un capteur de pression pour détecter la pression de gonflage du ballonet de dilatation et/ou du ballonet de régulation.

4. Cathéter (1) selon l'une quelconque des revendications précédentes, **caractérisé en ce que** ledit cathéter comprend un dispositif de commande pour commander le gonflage ou le dégonflage du ballonet de dilatation (2) et/ou du ballonet de régulation (3) par rapport à l'écoulement détecté d'un fluide et/ou par rapport à la pression détectée.

5. Cathéter (1) selon l'une quelconque des revendications précédentes, **caractérisé en ce que** les moyens de gonflage et/ou de dégonflage du ballonet de régulation (3) comprennent une pompe commandable entraînée automatiquement.

6. Cathéter (1) selon la revendication 5, **caractérisé en ce que** la pompe est commandée par rapport à la pression de gonflage détectée du ballonet de régulation.

7. Cathéter (1) selon l'une quelconque des revendications précédentes, **caractérisé en ce que** le cathéter comprend des moyens pour distribuer localement un médicament.

8. Cathéter (1) selon la revendication 7, **caractérisé en ce que** les moyens pour distribuer localement un médicament comprennent une lumière séparée dans le corps tubulaire ou un revêtement sur le ballonet ou une distribution par l'intermédiaire de pores dans le ballonet.
